(19) **Europäisches Patentamt**

**European Patent Office**

**Office européen des brevets**

(11) **EP 0 701 452 B1**

(12) **FASCICULE DE BREVET EUROPEEN**

(45) Date de publication et mention
de la délivrance du brevet:
**07.07.1999 Bulletin 1999/27**

(51) Int. Cl.$^6$: **A61L 9/04**

(86) Numéro de dépôt international:
**PCT/FR95/00430**

(21) Numéro de dépôt: **95916707.3**

(22) Date de dépôt: **05.04.1995**

(87) Numéro de publication internationale:
**WO 95/26757 (12.10.1995 Gazette 1995/43)**

(54) **PROCEDE DE DIFFUSION D'UNE SUBSTANCE ODORANTE**

VERFAHREN FUER DIE DIFFUSION EINES RIECHENDEN STOFFS

METHOD FOR DIFFUSING AN ODORIFEROUS SUBSTANCE

(84) Etats contractants désignés:
**AT BE CH DE DK ES FR GB GR IT LI LU MC NL PT SE**

(30) Priorité: **05.04.1994 FR 9403983**

(43) Date de publication de la demande:
**20.03.1996 Bulletin 1996/12**

(73) Titulaire:
**L'AIR LIQUIDE, SOCIETE ANONYME POUR L'ETUDE ET L'EXPLOITATION DES PROCEDES GEORGES CLAUDE**
**75321 Paris Cédex 07 (FR)**

(72) Inventeur: **BARBIER, Jean-Paul**
**F-78112 Forqueux (FR)**

(74) Mandataire:
**Le Moenner, Gabriel et al**
**Societé l'Air Liquide**
**Service Brevets et Marques**
**75, Quai d'Orsay**
**75321 Paris Cédex 07 (FR)**

(56) Documents cités:
**WO-A-89/03227          US-A- 4 603 030**

• **PATENT ABSTRACTS OF JAPAN vol. 13, no. 394 (C-631) 31 Août 1989 & JP,A,01 139 507 (HITACHI LTD) 1 Juin 1989**

Printed by Xerox (UK) Business Services
2.16.7/3.6

## Description

La présente invention a pour objet un procédé de diffusion d'une substance odorante, tel un parfum, ainsi qu'un mélange gazeux comprenant une substance odorante à l'état gazeux et un gaz inodore et respirable.

On connaît différents procédés usuels pour diffuser une substance odorante. Parmi ces procédés, on peut notamment citer ceux utilisant un mélange constitué d'une substance odorante à l'état solide ou liquide, dissoute dans un solvant liquide. La diffusion dans l'air de ce mélange permet la diffusion de la substance odorante. Un tel procédé peut être mis en oeuvre par exemple au moyen d'une bombe aérosol quand le solvant est suffisamment volatil ou en portant à ébullition la solution dans laquelle est dissoute ladite substance odorante. Cette diffusion peut être favorisée par entraînement des vapeurs de solvant par des courants d'air. Ceux-ci peuvent être naturels ou, pour une diffusion plus importante, créés artificiellement, par exemple au moyen d'un système de ventilation.

La diffusion de substance odorante peut notamment avoir pour but de masquer une odeur désagréable ou de répandre une odeur agréable ou fragrance. Récemment, on a utilisé des substances odorantes en vue de renforcer des odeurs naturelles dans un but marketing ou d'aide à la vente. Ainsi on a proposé de diffuser à proximité d'étals, par exemple une boulangerie ou une épicerie, des substances odorantes émettant une odeur de brioches, de pain frais ou de fruits. Dans ce cas, la diffusion de substance odorante a pour but principal d'attirer la clientèle.

Toutefois, les procédés usuels, mentionnés ci-dessus, visant à la diffusion de substances odorantes présentent des inconvénients lorsqu'on veut diffuser des substances odorantes à distance importante de leur source. En effet, des moyens onéreux et de maniement difficile, par exemple des ventilateurs, doivent être mis en oeuvre.

La présente invention a pour objet un procédé de diffusion de substances odorantes de mise en oeuvre simplifiée, ne nécessitant pas de moyens onéreux et permettant en outre de diffuser lesdites substances en des teneurs constantes et en des zones délimitées.

L'invention consiste alors en un procédé de diffusion d'une substance odorante caractérisée en ce que :

    a) on conditionne dans un récipient un mélange gazeux pressurisé à au moins 5 bar, ledit mélange gazeux comprenant la substance odorante à l'état gazeux et au moins un gaz inodore et respirable,
    b) on relie le récipient à un moyen pour libérer de manière contrôlée le mélange gazeux hors dudit récipient, et
    c) on libère de manière contrôlée le mélange gazeux hors du récipient, de sorte à permettre la diffusion de la substance odorante à l'état gazeux.

Le récipient est habituellement une bouteille de gaz, de préférence une bouteille de gaz comprimé. Le moyen permettant de libérer de manière contrôlée ledit mélange gazeux peut notamment consister en un détendeur ou un régulateur de débit comportant une vanne et un débitmètre.

Ledit mélange gazeux, libéré hors du récipient, peut diffuser, en un premier temps, dans une conduite ou un réseau de conduites, puis, dans un deuxième temps, à l'air libre. Lesdites conduites peuvent être par exemple de simples tuyaux en caoutchouc de faible diamètre. De part la pression à laquelle il est soumis, ledit mélange peut diffuser à distance du récipient qui le contient. Par ailleurs, lesdites conduites peuvent être utilisées comme moyen pour faire diffuser la substance odorante en une zone bien localisée. De ce fait, il est possible de ne consommer qu'une faible quantité dudit mélange pour produire l'odeur choisie en un endroit précis.

Selon un autre aspect de l'invention, le mélange gazeux peut diffuser dans un premier temps dans une enceinte, telle une cuve de stockage ou une conduite, contenant un tiers gaz, ce tiers gaz pouvant ultérieurement être libéré hors de l'enceinte pour être conduit vers un poste d'utilisation ou diffuser à l'air libre.

Ledit tiers gaz contenu dans l'enceinte permet de diluer le mélange gazeux libéré hors du récipient.

Le tiers gaz peut consister en un gaz quelconque, inodore ou peu odorant, auquel on veut conférer une odeur. De préférence, ce tiers gaz est un gaz respirable tel que défini ci-après, et plus préférentiellement, ce tiers gaz est de l'oxygène ou de l'air enrichi en oxygène, par exemple de l'air enrichi à plus de 25 % molaire en oxygène.

Un tel gaz riche en oxygène doit, dans certaines applications, pouvoir être détecté rapidement en vue d'éviter des incidents liés à une fuite de ce tiers gaz hors de l'enceinte qui le contient.

Une suroxygénation d'une zone ou d'un milieu peut en effet entraîner une activation des phénomènes de combustion, entraîner une dégradation de certains matériels comme des moteurs électriques.

Le procédé selon l'invention permet de faire diffuser rapidement et de manière homogène et simple la substance odorante dans le tiers gaz, sans en altérer substantiellement la composition.

Dans le cadre de la présente invention on entend par le terme "gaz respirable" un gaz ou une association de gaz qui peut être respiré par l'homme ou un mammifère dans des conditions telles que sa santé ne soit pas mis en danger.

Le gaz inodore et respirable selon l'invention est, de préférence, également non nocif, non toxique, non corrosif et ininflammable. De plus, il présente avantageusement peu ou pas de risque pour l'environnement.

C'est pourquoi l'air constitue un gaz respirable tout particulièrement préféré dans le cadre de l'invention.

Outre l'air, on peut également utiliser en tant que gaz inodore et respirable, de l'oxygène ou une association de gaz comprenant de l'air et/ou de l'oxygène et un gaz tel que l'azote, l'argon, l'hélium ou le protoxyde d'azote.

La substance odorante selon l'invention peut être un parfum ou tout autre substance fournissant une fragrance. De telles substances sont notamment décrites dans "Cosmetics, Fragrances and Flavors" de Louis Appel, Novox Inc. Publisher, Whiting, New Jersey 08759, pp 231-256, dont référence est intégrée dans la présente description.

La substance odorante peut être également malodorante, en vue de produire une odeur désagréable. Dans ce cas, le mélange gazeux est essentiellement destiné à diffuser dans un tiers gaz en vue de conférer une odeur à ce dernier, par exemple pour permettre d'en détecter les fuites. A titre de substance malodorante, on peut citer $H_2S$ ou les composés soufrés organiques comme le sulfure de diméthyle.

La concentration de la substance odorante à l'état gazeux dans ledit mélange gazeux doit préférentiellement être supérieure ou très supérieure à son seuil minimum de perception olfactive dans l'air $\theta'$. Ce seuil minimal de perception peut être déterminé selon la méthode décrite dans "Cosmetics, Fragrances and Flavors", mentionné ci-dessus. Selon cette méthode, on détermine le seuil minimal de perception de la substance dans l'air $\theta'$, exprimée en microgramme par litre d'air, au moyen de la formule suivante : $\theta' = 5,4$ M. $10^{-5} . \theta. P.$
dans laquelle :

- M représente la masse molaire de la substance odorante
- $\theta$ représente la concentration minimale de la substance odorante dans l'eau pour laquelle il y a détection olfactive (en ppm, soit en partie par million exprimée en masse).
- P est la tension de vapeur à 25° C (exprimée en mm de mercure) de la substance odorante. La valeur $\theta$ est déterminée par un panel de personnes extraînées à la détection olfactive.

Habituellement, une substance odorante pouvant être mise en oeuvre dans ledit mélange, présente un seuil de perception dans l'air compris entre 0,001 et 10 ppb vol., généralement compris entre 0,01 et 1 ppb (1 ppb vol. = une partie par milliard en volume, soit une partie pour $10^9$ en volume). La concentration de la substance odorante à l'état gazeux dans ledit mélange gazeux peut-être supérieure à 0,01 ppm vol. (1 ppm vol. = une partie par million en volume), et, de préférence, inférieure au seuil d'inflammabilité. Ainsi, la concentration en ladite substance odorante peut être inférieure à 2 % en volume quand le mélange gazeux est soumis à une pression de 80 bar, ou inférieure à 1 % en volume sous une pression de 200 bar.

Lorsque le mélange gazeux est destiné à diffuser dans un tiers gaz en vue de lui conférer une odeur comme indiqué ci-dessus, la concentration en la substance odorante dans le mélange gazeux est avantageusement choisie de sorte que la concentration en ladite substance odorante dans le tiers gaz soit comprise entre 0,1 et 5 ppm vol., de préférence entre 2,5 et 5 ppm vol.. Ces concentrations sont plus particulièrement mises en oeuvre quand la substance odorante est malodorante.

Lorsque la substance odorante est destiné à diffuser à l'air libre, après avoir ou non diffusé dans une ou plusieurs conduite, sa concentration dans le mélange gazeux est généralement comprise entre 0,01 et 1000 ppm vol., plus généralement comprise entre 0,1 et 100 ppm vol.

Quel que soit le mode de réalisation de l'invention, il importe, en général, que la teneur en la substance odorante libérée à l'air libre soit inférieure à son seuil de toxicité (valeur moyenne d'exposition pendant 8 heures / jour et 5 jours / semaine).

A titre d'exemple, des substances odorantes et les fragrances qu'elles émettent, convenant dans le cadre de la présente invention, sont mentionnées dans le tableau I ci-dessous.

TABLEAU I

| Odeur | Substance odorante | Masse molaire (M) | Tension de vapeur à 25° C P (mm Hg) | Concentration minimale θ de la substance dans l'eau peur laquelle il y a détection olfactive (ppm) | Seuil minimal de perception de la substance dans l'air : θ' | |
|---|---|---|---|---|---|---|
| | | | | | en microgramme par litre d'air | en ppb.vol. |
| Citron | Citral | 152 | 0,05 | 1 | $4,1.\,10^{-4}$ | 0.06 |
| Banane | acétate d'isoamyle | 130 | 5,6 | 0,1 | $3,9.10^{-3}$ | 0,6 |
| Rose | alcool phényléthylique | 122 | 0,054 | 1 | $3,5.10^{-4}$ | 0,06 |
| Fraise | éthylméthylphénylglycidate | 206 | 0,03 | 0,001 | $3,3.10^{-8}$ | $3,6.10^{-6}$ |
| Jasmin | acétate de benzyle | 150 | 0,120 | 1 | $9,7.10^{-4}$ | 0,15 |

Ledit mélange gazeux selon l'invention peut être pressurisé dans le récipient qui le contient à une pression comprise entre 50 et 200 bar, de préférence entre 100 et 200 bar.

Le mélange gazeux comprenant la substance odorante à l'état gazeux et au moins un gaz inodore et respirable, peut être préparé par tous moyens classiques et de fabrication des mélanges de gaz dont l'un au moins des constituants est présent en une teneur très supérieure à la teneur en au moins un autre constituant.

Un procédé de ce type peut par exemple consister à introduire un large excès de la substance odorante dans le récipient, puis à pressuriser ce dernier par le gaz inodore et respirable. La tension de vapeur de la substance odorante va alors se mélanger au gaz inodore et respirable pour former ledit mélange gazeux, qui peut alors être libéré hors du récipient. Le mélange gazeux ainsi obtenu, permet la diffusion de la substance odorante en une teneur croissante au cours du temps. En effet, au fur et à mesure de la libération du mélange gazeux hors du récipient, la pression partielle en gaz inodore et respirable dans le récipient diminue alors que celle en la substance odorante reste constante puisqu'elle a été introduite en large excès.

Selon un autre procédé, on forme ledit mélange au moyen d'une quantité déterminée de substance odorante. Cette quantité peut être choisie de sorte que ledit mélange, lorsqu'il est libéré hors du récipient, fournisse une concentration, et donc une odeur substantiellement constante et prédéterminée quelque soit la pression dudit mélange dans le récipient. La substance odorante est introduite avant, pendant ou après ledit gaz inodore et respirable.

En vue de maintenir constante la concentration de la substance odorante dans le récipient, il est toutefois préférable d'utiliser un gaz inodore et respirable débarrassé de l'eau qu'il pourrait contenir et de prétraiter la paroi interne du récipient en vue d'éliminer substantiellement tout phénomène d'adsorption de surface. Pour ce faire, on peut sécher le gaz inodore et respirable ; alternativement ou en plus on peut également nettoyer la paroi interne du récipient pour en éliminer substantiellement toute matière organique telle graisse, huile ou hydrocarbure, suite à quoi cette paroi est séchée, de préférence sous vide, pour substantiellement éliminer toute trace rémanente de composé, tel l'eau, susceptible d'adsorber la substance odorante.

Ce traitement de la paroi interne peut, bien entendu, être également effectué dans le cadre du premier procédé décrit ci-dessus selon lequel on introduit un large excès de la substance odorante dans le récipient.

Selon un autre aspect, l'invention concerne un mélange gazeux comprenant une substance odorante, telle que mentionnée plus haut, et un gaz inodore et respirable tel l'air ou une association de gaz respirable telle que définie plus haut, ce mélange gazeux étant conditionné dans un récipient sous une pression supérieure ou égale à cinq bar, de préférence comprise entre 100 et 200 bar.

Avantageusement, la paroi interne du récipient est prétraitée de la manière indiquée ci-dessus, afin de limiter ou de substantiellement éliminer les phénomènes d'adsorption de surface de la substance odorante. Un tel prétraitement peut consister en un nettoyage suivi d'un séchage minutieux, de préférence sous vide, de la paroi interne.

Les exemples qui suivent ont pour but d'illustrer la présente invention.

EXEMPLE 1 :

Mélange gazeux pour la diffusion d'une odeur de banane à une concentration déterminée

On injecte au moyen d'une seringue, dans une bouteille de capacité de 50 litres préalablement nettoyée et chauffée sous vide, 2 grammes d'acétate d'isoamyle dont la tension de vapeur à 25°C est de 5,6 mm de mercure (746 Pa). On introduit ensuite de l'air sec, d'une teneur en eau inférieure à 5 ppm vol, jusqu'à une pression de 200 bar. On forme ainsi une composition de 36 ppm vol. d'acétate d'isoamyle dans l'air.
L'acétate d'isoamyle est une substance développant une odeur de banane avec un seuil de perception de 0,6 ppb.vol. calculé selon la méthode décrite par Louis Appell dans "Cosmetic, Fragrance and Favors", Novox Inc. Publisher Whiting, New Jersey 08759, pp. 231-256. La concentration en acétate d'isoamyle dans la composition est donc 60.000 fois supérieure à son seuil de perception, ce qui permet de détecter facilement l'odeur de banane par diffusion de ce mélange.

EXEMPLE 2 :

Mélange gazeux pour la diffusion d'une odeur de banane à des concentrations augmentant lorsque la pression dans la capacité le contenant diminue

On injecte au moyen d'une seringue, dans une bouteille de capacité 50 litres préalablement nettoyée et chauffée sous vide, environ 200 grammes d'acétate d'isoamyle. On introduit ensuite de l'air jusqu'à une pression de 200 bar. On forme ainsi une composition de 36 ppm vol. d'acétate d'isoamyle dans l'air à 200 bar. Cette concentration augmente au fur et à mesure de la vidange de la bouteille pour atteindre jusqu'à environ 0,7 % volume, lorsque celle-ci est à la pression atmosphérique.

EXEMPLE 3 :

Mélange gazeux pour la diffusion d'une odeur de rose à une concentration déterminée

On injecte au moyen d'une seringue, dans une capacité de 50 litres préalablement nettoyée et chauffée sous vide, 0,02 gramme d'alcool phényléthylique dont la tension de vapeur à 25°C est de 0,054 mm de mercure (7,2 Pa). On introduit ensuite de l'air très sec (moins de 1 ppm d'eau) jusqu'à une pression de 200 bar. on forme ainsi une composition de 0,35 ppm vol. d'alcool phényléthylique dans l'air.
L'alcool phényléthylique est une substance développant une odeur de rose avec un seuil de perception de 0,06 p.p.b. vol. calculé selon la méthode décrite par Louis Appel dans le même ouvrage que celui cité précédemment. La concentration en alcool phényléthylique dans la composition est donc presque 6.000 fois supérieure à son seuil de perception ce qui permet de détecter l'odeur de rose par diffusion de ce mélange.

EXEMPLE 4 :

Mélange gazeux pour la diffusion d'une odeur de rose à des concentration augmentant lorsque la pression dans le récipient le contenant diminue

On injecte au moyen d'une seringue, dans une bouteille de capacité 50 litres préalablement nettoyée et chauffée sous vide, plus de deux grammes d'alcool phényléthylique. On introduit ensuite de l'air jusqu'à une pression de 200 bar. on forme ainsi une composition de 0,35 ppm vol. d'alcool phényléthylique dans l'air à 200 bar. Cette concentration augmente au fur et à mesure de la vidange de la capacité pour atteindre jusqu'à 70 ppm vol. lorsque cette dernière est à la pression atmosphérique.

EXEMPLE 5 :

Diffusion d'une odeur de banane dans l'air

Le récipient contenant le mélange gazeux tel que préparé dans l'exemple 1 est disposé dans une pièce dont le volume est de 100m$^3$, dans laquelle sont présentes en moyenne au même moment cinq personnes et dont le renouvellement d'air est de 30m$^3$ par heure et par personne (comme l'exige la réglementation française).

Le récipient est muni d'un régulateur de pression, d'un régulateur de débit comportant une vanne et un débitmètre, et d'un tuyau permettant de délivrer le mélange gazeux en un endroit choisi de la pièce.

On règle le débitmètre de sorte que la concentration de la substance odorante dans la pièce soit 30 fois supérieure à son seuil de perception, soit 18 ppb vol.. On maintient cette concentration en réglant le débit à 75 l/h. Ce débit peut être maintenu constant pendant environ 133 heures.

**Revendications**

1. Procédé de diffusion d'une substance odorante, caractérisé en ce que :

   a) on conditionne dans un récipient un mélange gazeux pressurisé à au moins 5 bar, ledit mélange comprenant la substance odorante à l'état gazeux et au moins un gaz inodore et respirable,
   b) on relie le récipient à un moyen pour libérer de manière contrôlée le mélange gazeux hors dudit récipient, et
   c) on libère de manière contrôlée ledit mélange gazeux hors du récipient, de sorte à permettre la diffusion de la substance odorante à l'état gazeux.

2. Procédé selon la revendication 1, caractérisé en ce que le récipient est une bouteille de gaz.

3. Procédé selon l'une des revendications 1 et 2, caractérisé en ce qu'avant de conditionner ledit mélange dans le récipient, la paroi interne de celui est traitée de sorte à substantiellement éliminer les phénomènes d'adsorption de surface.

4. Procédé selon l'une des revendications 1 à 3, caractérisé en ce que ledit mélange gazeux, libéré hors du récipient, diffuse, dans un premier temps, dans une conduite ou un réseau de conduites, puis, dans un deuxième temps, à l'air libre.

5. Procédé selon l'une des revendications 1 à 3, caractérisé en ce que ledit mélange gazeux, libéré hors du récipient, diffuse directement à l'air libre.

6. Procédé selon l'une des revendications 1 à 3, caractérisé en ce que ledit mélange gazeux diffuse dans un tiers gaz, contenu dans un récipient et confère une odeur à ce tiers gaz.

7. Procédé selon l'une des revendications 1 à 6, caractérisé en ce que le gaz inodore et respirable est également non nocif, non toxique, non corrosif et ininflammable.

8. Procédé selon la revendication 7, caractérisé en ce que le gaz inodore et respirable est l'air.

9. Procédé selon l'une des revendications 1 à 8, caractérisé en ce que la substance odorante est une substance fournissant une fragrance .

10. Procédé selon l'une des revendications 1 à 8, caractérisé en ce que la substance odorante est une substance fournissant une odeur désagréable.

11. Procédé selon l'une des revendications 1 à 10, caractérisé en ce que ledit mélange est pressurisé à une pression comprise entre 50 et 200 bar.

12. Procédé selon l'une des revendications 1 à 11, caractérisé en ce que la concentration de la substance odorante à l'état gazeux dans ledit mélange est supérieure à 0,01 ppm vol., généralement comprise entre à 0,01 ppm vol et 2%, plus généralement comprise entre 0,1 et 100 ppm vol.

13. Un mélange gazeux comprenant une substance odorante à l'état gazeux et un gaz inodore et respirable, caractérisé en ce que ledit mélange gazeux est conditionné dans un récipient sous une pression supérieure ou égale à 5 bar, de préférence comprise entre 50 et 200 bar.

14. Un mélange selon la revendication 13, caractérisé en ce que la paroi interne du récipient est prétraitée de sorte à substantiellement éliminer les phénomènes d'absorption de la substance odorante par la surface interne du récipient.

**15.** Un mélange selon l'une des revendications 13 et 14 caractérisé en ce que le gaz inodore et respirable est l'air.

**16.** Utilisation d'un mélange gazeux selon l'une des revendications 13 à 15, pour odoriser un tiers gaz contenu dans une enceinte, de sorte à permettre de détecter des fuites du tiers gaz hors de l'enceinte qui le contient.

## Claims

**1.** Process for diffusing an odoriferous substance, characterized in that:

   a) a gas mixture pressurized to at least 5 bar is placed in a container, the said mixture comprising the odoriferous substance in gas form and at least one odourless and breathable gas,
   b) the container is connected to a means for releasing the gas mixture outside the said container in a controlled manner, and
   c) the said gas mixture is released in a controlled manner outside the container, so as to allow diffusion of the odoriferous substance in gas form.

**2.** Process according to Claim 1, characterized in that the container is a gas bottle.

**3.** Process according to either of Claims 1 and 2, characterized in that before placing the said mixture in the container, the inner wall of this container is treated so as to substantially eliminate any phenomena of surface adsorption.

**4.** Process according to one of Claims 1 to 3, characterized in that the said gas mixture, released outside the container, diffuses, in a first stage, in a duct or a network of ducts, and then, in a second stage, in free air.

**5.** Process according to one of Claims 1 to 3, characterized in that the said gas mixture, released outside the container, diffuses directly in free air.

**6.** Process according to one of Claims 1 to 3, characterized in that the said gas mixture diffuses in a third gas, contained in a container, and confers an odour to this third gas.

**7.** Process according to one of Claims 1 to 6, characterized in that the odourless and breathable gas is also non-harmful, non-toxic, non-corrosive and non-flammable.

**8.** Process according to Claim 7, characterized in that the odourless and breathable gas is air.

**9.** Process according to one of Claims 1 to 8, characterized in that the odoriferous substance is a substance giving fragrance.

**10.** Process according to one of Claims 1 to 8, characterized in that the odoriferous substance is a substance giving an offensive odour.

**11.** Process according to one of Claims 1 to 10, characterized in that the said mixture is pressurized to a pressure of between 50 and 200 bar.

**12.** Process according to one of Claims 1 to 11, characterized in that the concentration of the odoriferous substance in gas form in the said mixture is greater than 0.01 ppm vol., generally between 0.01 ppm.vol and 2%, more generally between 0.1 and 100 ppm.vol.

**13.** Gas mixture comprising an odoriferous substance in gas form and an odourless and breathable gas, characterized in that the said gas mixture is placed in a container under a pressure of greater than or equal to 5 bar, preferably between 50 and 200 bar.

**14.** Mixture according to Claim 13, characterized in that the inner wall of the container is pretreated in order to substantially eliminate any phenomena of absorption of the odoriferous substance by the inner surface of the container.

**15.** Mixture according to either of Claims 13 and 14, characterized in that the odourless and breathable gas is air.

**16.** Use of a gas mixture according to one of Claims 13 to 15, to give an odour to a third gas contained in an enclosure,

EP 0 701 452 B1

so as to permit the detection of leaks of the third gas from the enclosure containing it.

**Patentansprüche**

1.  Verfahren zum Verströmen eines Geruchsstoffs, dadurch gekennzeichnet, daß man

    a) eine gasförmige Mischung, die unter einem Druck von mindestens 5 bar steht, in ein Behältnis verpackt, wobei diese Mischung den Geruchsstoff im gasförmigen Zustand sowie mindestens ein geruchloses und atembares Gas enthält,
    b) das Behältnis an ein Element zur kontrollierten Freisetzung der gasförmigen Mischung aus diesem Behältnis anschließt sowie
    c) die gasförmige Mischung aus dem Behältnis auf eine kontrollierte Weise freisetzt, die ein Verströmen des Geruchsstoffs im gasförmigen Zustand gestattet.

2.  Verfahren nach Anspruch 1, dadurch gekennzeichnet, daß es sich bei dem Behältnis um eine Gasflasche handelt.

3.  Verfahren nach einem der Ansprüche 1 und 2, dadurch gekennzeichnet, daß, bevor die Mischung in dem Behältnis verpackt wird, die Innenwand des letzteren so behandelt wird, daß Oberflächenadsorptionserscheinungen im wesentlichen beseitigt werden.

4.  Verfahren nach einem der Ansprüche 1 bis 3, dadurch gekennzeichnet, daß die aus dem Behältnis freigesetzte gasförmige Mischung erst in einen Gang oder ein Gangsystem und dann schließlich in die freie Luft verströmt wird.

5.  Verfahren nach einem der Ansprüche 1 bis 3, dadurch gekennzeichnet, daß die aus dem Behältnis freigesetzte gasförmige Mischung direkt in die freie Luft verströmt wird.

6.  Verfahren nach einem der Ansprüche 1 bis 3, dadurch gekennzeichnet, daß die gasförmige Mischung in ein drittes Gas, das in einem Behältnis vorliegt, verströmt wird und diesem dritten Gas einen Geruch verleiht.

7.  Verfahren nach einem der Ansprüche 1 bis 6, dadurch gekennzeichnet, daß das geruchlose und atembare Gas außerdem harmlos, nichttoxisch, nichtkorrodierend und flammwidrig ist.

8.  Verfahren nach Anspruch 7, dadurch gekennzeichnet, daß es sich bei dem geruchlosen und atembaren Gas um Luft handelt.

9.  Verfahren nach einem der Ansprüche 1 bis 8, dadurch gekennzeichnet, daß es sich bei dem Geruchsstoff um eine Substanz handelt, die einen Wohlgeruch verbreitet.

10. Verfahren nach einem der Ansprüche 1 bis 8, dadurch gekennzeichnet, daß es sich bei dem Geruchsstoff um eine Substanz handelt, die einen unangenehmen Geruch verbreitet.

11. Verfahren nach einem der Ansprüche 1 bis 10, dadurch gekennzeichnet, daß die Mischung unter einem Druck zwischen 50 und 200 bar steht.

12. Verfahren nach einem der Ansprüche 1 bis 11, dadurch gekennzeichnet, daß die Konzentration des Geruchsstoffs im gasförmigen Zustand in der Mischung über 0,01 Vol.-ppm, im allgemeinen zwischen 0,01 Vol.-ppm und 2%, noch allgemeiner zwischen 0,1 und 100 Vol.-ppm beträgt.

13. Gasförmige Mischung, die einen Geruchsstoff im gasförmigen Zustand sowie ein geruchloses und atembares Gas enthält, dadurch gekennzeichnet, daß die gasförmige Mischung unter einem Druck von 5 bar oder darüber, vorzugsweise zwischen 50 und 200 bar, in einem Behältnis verpackt ist.

14. Mischung nach Anspruch 13, dadurch gekennzeichnet, daß die Innenwand des Behältnisses so vorbehandelt ist, daß Absorptionserscheinungen des Geruchsstoffs an der Innenwand des Behältnisses im wesentlichen beseitigt werden.

15. Mischung nach einem der Ansprüche 13 und 14, dadurch gekennzeichnet, daß es sich bei dem geruchlosen und atembaren Gas um Luft handelt.

8

16. Verwendung einer gasförmigen Mischung nach einem der Ansprüche 13 bis 15 zur Verleihung eines Geruchs an ein drittes Gas, das in einem abgeschlossenen Raum vorliegt, so daß ein Ausströmen des dritten Gases aus dem abgeschlossenen Raum, der es enthält, nachgewiesen werden kann.